# EUROPÄISCHE PATENTSCHRIFT

(11) **EP 0 770 388 B1**
(45) Veröffentlichungstag und Bekanntmachung des Hinweises auf die Patenterteilung: **25.03.1998**
(21) Anmeldenummer: 96116919.0
(22) Anmeldetag: 22.10.1996
(51) Int. Cl.: A61K 31/57

(54) **Mehrphasenpräparat zur Kontrazeption auf der Basis natürlicher Estrogene**
Multiphase contraceptive preparation based on natural estrogens
Préparation contraceptive à plusieures phases, basée sur des oestrogènes naturels

(30) Priorität: 28.10.1995 DE 19540253
(43) Veröffentlichungstag der Anmeldung: 02.05.1997
(73) Patentinhaber: Jenapharm GmbH & Co. KG, 07745 Jena (DE)
(72) Erfinder: Dittgen, Michael, Prof. Dr., 99510 Apolda (DE); Fricke, Sabine, Dr., 07749 Jena (DE); Hoffmann, Herbert, Prof. Dr., 07749 Jena (DE); Moore, Claudia, Dr., 07747 Jena (DE); Oettel, Michael, Prof. Dr., 07743 Jena (DE); Ostertag, Monika, 37073 Göttingen (DE)

(56) Entgegenhaltungen:
- EP-A- 0 491 438
- DE-A- 3 229 612
- DE-A- 4 308 406
- DE-A- 4 344 462
- DE-A- 4 429 374
- US-A- 4 292 315

## Beschreibung

Die Erfindung betrifft ein Mehrphasenpräparat zur Kontrazeption auf der Basis natürlicher Estrogene.

Orale Kontrazeptiva kamen erstmals in den frühen 60er Jahren auf den Markt. Durch fortlaufende Forschungsarbeiten gelang es, die notwendigen Dosierungen an Hormonen schrittweise zu reduzieren. Heute existieren niedrig dosierte orale Kontrazeptiva, die hauptsächlich aus einer Estrogenkomponente und einer Gestagenkomponente bestehen. Dabei werden die Hormongaben in unterschiedlichsten Kombinationen und Dosierungen in Form von Kombinationspräparaten (Einphasenpräparaten) oder abgestuften Kombinationspräparaten (Stufenpräparaten) sowie Sequenzpräparaten (Zweiphasenpräparaten) über einen Zeitraum von 21 bzw. 28 Tagen verabreicht.

Einphasenpräparate (üblicherweise als Kombinationspräparate bezeichnet) sind gekennzeichnet durch eine gleichbleibende Dosis des jeweiligen Estrogens und Gestagens in jeder Tagesdosis. Durch die gleichzeitige Gabe der Gestagenkomponente mit dem Estrogenanteil vom ersten Applikationstag an, entfalten Kombinationspräparate eine hohe kontrazeptive Sicherheit.

Bei allen Formen der Kombinationspräparate wird der ovulatorische LH-Gipfel zuverlässig supprimiert, so daß sowohl Ovulation als auch Corpus-luteum-Bildung unterdrückt werden (ELSTEIN, M. et al.: Studies on low dose oral contraceptives: cervical mucus and plasma hormone changes in relation to circulating d-norgestrel and 17-ethinyl estradiol concentrations. Fertil Steril 27: 892 (1976); Kontrazeption mit Hormonen. Hrsg. Taubert, H.-D. und Kuhl, H., Georg Thieme Verlag Stuttgart/New York, (1995), S. 125-128).
Allerdings kann unter dem Einfluß eines Gestagens die frühere sekretorische Umwandlung des noch wenig entwickelten Endometriums das Auftreten von Zwischenblutungen vor allem innerhalb der ersten Zyklen zur Folge haben.

Modifizierte Kombinations- oder Stufenpräparate gibt es bislang als 2-Stufenpräparate und als 3-Stufenpräparate.
Als 2-Stufenpräparate werden jene bezeichnet , die in der ersten Einnahmephase eine gegenüber den konventionellen Kombinationspräparaten verminderte Gestagendosis enthalten, die in der zweiten Phase (Zyklushälfte) erhöht wird. Bei einem 21/22-tägigen Pillenregime wird die niedrige Gestagendosis 11 Tage verabfolgt, während die Estrogendosis über den Einnahmezeitraum gleich bleibt.

3-Stufenpräparate enthalten in den ersten Einnahmetagen neben niedrigdosierten Estrogenen niedrigdosierte Gestagene, die in 2 Stufen zur höchsten Gestagendosierung in den letzten 7 bis 10 Tagen ansteigen, während der Estrogenanteil entweder gleichbleibt oder mittelzyklisch kurzfristig über die Dauer von 5 bis 6 Tagen in Analogie zum normalen physiologischen Zyklus erhöht wird. 3-Stufenpräparate bieten die Möglichkeit, die Gestagendosis an applizierten Gestagen niedriger zu halten als bei anderen oralen Kontrazeptiva (CARLBORG, L.: Comparison of contraceptive acceptability of levornorgestrel and ethinyl oestradiol administered in one three-phasic (Trionette) and one monophasic (Neolette) version. Contraception, 27:5 (1983)).

In der Patentschrift DE 43 39 934 C2 wird ein gattungsmäßiges ovulationshemmendes Mittel zur hormonalen Kontrazeption aufgezeigt, bestehend aus einer Kombination von mehreren Estrogenen und einem Gestagen, wobei die Tageseinheiten sowohl eine gleichbleibende als auch eine gestaffelte Estrogen- und Gestagendosis und ein einnahmefreies Intervall über die Zyklusdauer aufweisen.
Die Patentschrift EP 0491 415 B1 beschreibt eine mehrphasige Kombination und ein empfängnisverhütendes Set, in welcher/welchem innerhalb von drei Phasen über einen Zyklus und 4 Tagen einnahmefreies Intervall eine gestaffelte Estrogen- und Gestagendosierung verabreicht wird.

Zweiphasenpräparate (Sequenzpräparate) enthalten in den ersten 7 bis maximal 11 Tagen der Anwendung eine reine Estrogenkomponente und erst in den nachfolgenden 10 bis maximal 14 Tagen zusätzlich einen Gestagenanteil. Hierdurch unterliegt das Endometrium Veränderungen, die dem physiologischen Zyklusablauf sehr nahe kommen. Sie bewirken somit eine gute Zykluskontrolle.
Sequenzpräparate senken die basalen Gonadotropinspiegel in ähnlicher Weise wie Kombinationspräparate, wobei die FSH-Spiegel offensichtlich stärker unterdrückt sind als IH-Spiegel (AKTORIES, K. et al.: Die Beieinflussung des Ovarialzyklus durch verschiedene Typen hormonaler Kontrazeptiva. Geburtshilfe Frauenheilkunde 36: 318(1976)).

In den Patentschriften DE 41 04 385 C1 und DE 42 24 534 A1 werden Sequenzpräparate zur hormonalen Kontrazeption ohne einnahmefreies Intervall beschrieben, wobei die natürliche Estrogenkomponente über die Gesamtdauer des Zyklus verabreicht wird.
Nachteilig an diesen Lösungen ist, daß die Tagesdosiseinheiten mit relativ hohen Estrogendosierungen (teilweise 4 mg Estradiol/Tag bzw. Estradiol-Äquivalente/Tag) verabreicht werden.

Aus der Patentschrift US 4.921.843 ist ein Kontrazeptionssystem sowohl mit synthetischen als auch mit natürlichen Estrogenen bekannt, bei dem zwischen der Einnahme der letzten Hormon-Tagesdosiseinheit der zweiten Hormonkomponente eine einnahmefreie Zeit von mindestens einem Tag vorgesehen ist, ehe die neue Tagesdosiseinheit der ersten Hormonkomponente verabreicht wird. Dieses einnahmefreie Intervall soll vorzugsweise durch ein Placebo überbrückt werden.
Der Nachteil dieser Lösung besteht darin, daß dieses Sequenzpräparat hinsichtlich der kontrazeptiven Sicherheit etwas weniger zuverlässig ist, da während der reinen Estrogenphase beispielsweise fünf Tage lang 20 bis 40 µg Ethinylestradiol oder eine Dosierung eines natürlichen Estrogens, die der angegebenen Menge Ethinylestradiol entspricht, verabreicht werden. Damit wird die Ovulation nicht bei allen Frauen verhindert, zumal die peripheren kontrazeptiven Effekte des Gestagens fehlen. Es ist bekannt, daß die Ovulationshemmdosis täglich 100 µg Ethinylestradiols bedarf.

Die Patentschrift DE 43 08 406 C1 beschreibt ein mehrstufiges Kontrazeptivum mit sowohl 28 Tagesdosiseinheiten als auch hormonfreiem Intervall, wobei mindestens eine Stufe die Kombination von drei Komponenten, nämlich einem biogenen, einem synthetischen oder einem Gestagen enthält.

TAUBERT, H.-D. und KUHL, H. (Kontrazeption mit Hormonen, Hrsg. Taubert, H.-D. et al., Georg Thieme Verlag Stuttgart/New York, (1995), S. 397) Zeigen auf, daß der Einsatz des natürlichen Estradiols als Estrogenkomponente in oralen Kontrazeptiva fehlgeschlagen ist.
Während die Gestagenkomponente allein für einen zuverlässigen kontrazeptiven Schutz sorgt, ist die Proliferation des Endometriums durch das natürliche Estrogen unzureichend. Es resultieren Blutungsanomalien wie Zwischenblutungen bis hin zu blutungsfreien Zyklen.
TAUBERT, H.-D. und KUHL, H. betonen, daß ein Ausweg ein Einnahmeschema mit kontinuierlicher Estrogeneinnahme sein könnte.

An Versuchen zur Entwicklung und Wirksamkeit einer sogenannten 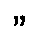 seminatürlichen Pille" auf Basis natürlicher Estrogene (z.B. 17β-Estradiol mikronisiert, Ester des natürlichen 17β-Estradiols, konjugierte Estrogene, Phytoestrogene und Estron, Estriol und deren Derivate) hat es bisher nicht gemangelt. Beispiele hierfür sind die Kombination Estradiol (Estradiolvalerat 1 mg bzw. 2 mg für 10 bzw. 11 Tage) und Cyproteronacetat (1 mg bzw. 2 mg für 10 bzw. 11 Tage) mit 33 % Zwischenblutungen (HIRVONEN, E. et al.: Oral contraceptive containig natural estradiol for premenopausal women. Maturitas 21:27, (1995) oder die Kombination mikronisiertes Estradiol (1 mg) und Desogestrel (0,150 mg) über 21 Tage mit unakzeptablen Blutungsstörungen (WENZEL, R. et al.:Ovulation inhibition with a combined oral contraceptive containing 1 mg micronized 17β-estradiol. Fertility and Sterility 60:4,( 1993) S. 616 - 619 und SERUP, J. et al.: Natural oestrogens for oral contraception. Lancet (1979, 01.09.),S. 471 - 472).

Im Vorfeld dieser Erfindung wurden Untersuchungen durchgeführt, die zum Ziel hatten, mit gleichbleibender Estradioldosierung über 28 Tage und gleichbleibender Gestagendosierung über 24 Tage sowie mit unterschiedlicher Estradioldosierung (2,0 mg, 4,0 mg und 2,0 mg über 7, 14 und 7 Tage) und gleichbleibender Gestagendosierung über 21 Tage eine akzeptable Zykluskontrolle zu erzielen. Die Zwischenblutungsrate von 25 % unterschieden sich nicht wesentlich von der in den bereits zitierten Studien von HIRVONEN und WENZEL und SERUP.

In der Patentschrift DE 44 29 374 C1 ist ein pharmazeutisches Präparat zur Kontrazeption und zur Hormonsubstitution mit biogener Estrogenkomponente ohne einnahmefreies Intervall aufgezeigt, wobei in der ersten und dritten Stufe ausschließlich biogene Estrogenkomponenten verabreicht werden und die zweite Stufe mindestens eine biogene Estrogenkomponente und mindestens eine Gestagenkomponente aufweist.
Neben dem Vorteil der Verabreichung von natürlichen Estrogenkomponenten besitzt diese Lösung den Nachteil, daß im Verlaufe des Zyklus eine insgesamt höhere Dosis eines Gestagens verabreicht wird.

Der Erfindung liegt die Aufgabe zugrunde, ein Mittel zur hormonalen Kontrazeption auf Basis natürlicher Estrogene aufzuzeigen, welches im Vergleich zu den gattungsgemäßen herkömmlichen ovulationshemmenden Mitteln bei niedriger Gestagendosierung und niedriger Estrogendosierung über die gesamte Dauer des Zyklus eine hohe kontrazeptive Sicherheit realisiert, das zyklische Blutungsverhalten verbessert und Nebenwirkungen, wie kanzerogene Potenz, Kumulation von Xenobiotika minimiert bzw. ausschließt.

Diese Aufgabe wird erfindungsgemäß durch ein Mehrphasenpräparat zur Kontrazeption gelöst,
- dessen erste Phase aus 2 bis 4 Tagesdosiseinheiten besteht und jede Tagesdosiseinheit als Wirkstoff ausschließlich natürliche Estrogene enthält,
- dessen zweite Phase aus 2 Gruppen von Tagesdosiseinheiten ei ner Kombination aus mindestens einem natürlichen Estrogen und mindestens einem synthetischen oder natürlichen Gestagen besteht, wobei die erste Gruppe aus 5 bis 3 Tagesdosiseinheiten und die zweite Gruppe aus 17 bis 13 Tagesdosiseinheiten gebildet wird
- dessen dritte Phase aus 2 bis 4 Tagesdosiseinheiten besteht und jede Tagesdosiseinheit als Wirkstoff ausschließlich natürliche Estrogene enthält
- wobei die Tagesdosiseinheit an natürlichem Estrogen innerhalb der Phasen konstant bleibt, jedoch von Phase 1 zu Phase 3 abfällt
- und der Anteil an synthetischen oder natürlichen Gestagen in der zweiten Gruppe der zweiten Phase den Anteil in der ersten Gruppe übersteigt
- dessen abschließende Phase aus 2 bis 4 Tagesdosiseinheiten be steht und jede Tagesdosiseinheit als Wirkstoff ein pharmazeutisch unbedenkliches Placebo enthält.

Vorteilhafte Ausgestaltungen der Erfindung sind im Anspruch 2 und 3 dargelegt.

Vorzugsweise beträgt der Gestagengehalt in den Tagesdosen der zweiten Gruppe der zweiten Phase das 1,5- bis 3fache des Gestagengehaltes in den Tagesdosen der ersten Gruppe.
Die Gesamtzahl der verabreichten Tagesdosiseinheiten ist dabei vorzugsweise 28. Als natürliche Estrogene eignen sich vor allem Estradiol wie z.B. 17β-Estradiol, Estradiolverbindungen wie z.B. 17β-estradiolvalerat, aus denen nach Einnahme im Körper Estradiol gebildet wird, konjugierte equine Estrogene sowie Phytestrogene. Als Gestagene werden vorzugsweise 19-Nortestosteron-Derivate wie Desogestrel, Dienogest, Gestoden und Levonorgestrel, C-21-Gestagene wie z.B. Medroxyprogesteronacetat, Chlarmadinonacetat sowie natürliches Progesteron angewandt.

Das erfindungsgemäße Mehrphasenpräparat ist besonders zur oralen Applikation geeignet, aber auch intravaginale, parenterale, incl. topische, rektale, intranasale, intrabukkale oder sublinguale Applikationen sind als Darreichungsformen möglich.

Das Mehrphasenpräparat wird mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt.
Für die orale Applikation kommen vorzugsweise Tabletten, Filmtabletten, Dragees oder Hartgelatinekapseln zur Anwendung.

Das erfindungsgemäße Mehrphasenpräparat zur Kontrazeption realisiert, daß in der ersten Zyklushälfte die Estrogen-Gestagen-Balance weit zugunsten der Estrogen-Komponente verschoben und in einer bestimmten Phase sogar auf den Gestageneinsatz völlig verzichtet wird.

Überraschenderweise gewährleistet dieses Regime auch einen Verzicht auf extrem hohe Estrogendosierungen (mehr als 4 mg Estradiol-Äquivalente/Tag) und Gestagendosierungen im Verlaufe des Zyklus.
Es wird bei niedriger Estrogendosierung die kontrazeptive Sicherheit der Estrogen/Gestagen-Kombination realisiert, obwohl phasenweise die peripheren kontrazeptiven Effekte des Gestagens minimiert sind bzw. fehlen.
Die Verkürzung des einnahmefreien Intervalls auf 2 bis 4 Tage erhöht sowohl die Zyklusstabilität als auch den kontrazeptiven Schutz. Die Verlängerung der Estrogenphase am Ende der Gestagenphase um 2 bis 4 Tage nimmt keinen Einfluß auf das regelmäßige Eintreten einer Entzugsblutung, verhindert jedoch zusätzlich das bereits beginnende Follikelwachstum für den nachfolgenden Zyklus.
Letzteres konnte in entsprechenden Untersuchungen mit der Kombination Estradiol und Dienogest nachgewiesen werden. Hierbei wurde die Größe der sonographisch gemessenen ovariellen Follikel von 10 mm niemals überschritten.
Tabelle 1 zeigt die Gesamtmenge der Estrogendosierung und der Gestagendosierung über die gesamte Zyklusdauer anhand der Beispiele 3 (konjugierte equine Estrogene/Desogestrel) und 5 (Estradiolvalerat/Dienogest) im Vergleich zu einem Applikationsregime über 28 Tage Hormongabe - entnommen der Patentschrift DE 44 29 374 C1, Beispiel 3 ( Estradiolvalerat/Dienogest), mit x bezeichnet - auf.

| | Estrogengehalt an natürlichen Estrogen [mg] | Gestagengehalt - Dienogest -[mg] |
|---|---|---|
| Beispiel 3 (x) | 56,0 | 52,0 |
| Beispiel 3 | 33,7 | 36,0 |
| Beispiel 5 | 51,0 | 36,0 |

Tabelle 2 zeigt die Estrogendosierung und die Gestagendosierung mittels Tagesdosiseinheiten über die gesamte Zyklusdauer ebenfalls anhand der Beispiele 3 (konjugierte equine Estrogene/Desogestrel) und 5 (Estradiolvalerat/Dienogest) im Vergleich zum Applikationsregime über 28 Tage Hormongabe - entnommen der Patentschrift DE 44 29 374 C1, Beispiel 3 ( Estradiolvalerat/Dienogest), mit x bezeichnet - auf.

| *Beispiel 3 (x)* | *Tage* | | | | | | |
|---|---|---|---|---|---|---|---|
| | *1-3* | *4* | *5-7* | *8-11* | *12-23* | *24-25* | *26-28* |
| Estrogengehalt - E [mg] | 1,5 | 1,5 | 1,5 | 2,5 | 2,5 | 2,5 | 1,5 |
| Gestagengehalt - G [mg] | - | - | 2,0 | 2,0 | 2,0 | 2,0 | - |

| *Beispiel 3* | | | | | | | |
|---|---|---|---|---|---|---|---|
| - E [mg] | 2,5 | 1,25 | 1,25 | 1,25 | 1,25 | 0,6 | - |
| - G [mg] | - | 1,0 | 1,0 | 2,0 | 2,0 | - | - |

| *Beispiel 5* | | | | | | | |
|---|---|---|---|---|---|---|---|
| - E [mg] | 3,0 | 2,0 | 2,0 | 2,0 | 2,0 | 1,0 | - |
| G [mg] | - | 1,0 | 1,0 | 2,0 | 2,0 | - | - |

Aus Tabelle 1 und Tabelle 2 ist ersichtlich, daß das erfindungsgemäße Applikationsregime im Vergleich zum herkömmlichen Applikationsregime die kontrazeptive Sicherheit gewährleistet durch eine niedrige gestaffelte Estrogendosierung und eine niedrige gestaffelte Gestagendosierung im Verlaufe des Zykluses.
Die die kontrazeptive Sicherheit gewährleistende Gestagendosierung ist beim herkömmlichen Applikationsregime nicht gestaffelt und verhältnismäßig hoch.

Die Erfindung soll an einigen Anwendungsbeispielen demonstriert werden.
Dabei wird insbesondere die Verbesserung des zyklischen Blutungsverhaltens der Frau nachgewiesen.

### Anwendungsbeispiel 1

Folgendes Regime kam zur Anwendung:
- Tage 1 bis 3: 3 mg Estradiolvalerat/d
- Tage 4 bis 7: 2 mg Estradiolvalerat/d + 0,075 mg Desogestrel/d
- Tage 8 bis 23: 2 mg Estradiolvalerat/d + 0,150 mg Desogestrel/d
- Tage 24 bis 25: 1 mg Estradiolvalerat/d
- Tage 26 bis 28: Placebo

Die Studie wurde an 101 Probandinnen im Alter von 18 bis 25 Jahren durchgeführt. Die Einnahmedauer betrug jeweils 6 Zyklen. Die durchschnittliche Rate der Zwischenblutungen (Durchbruchsblutungen und Spotting) sank von 20,2 % im ersten Einnahmezyklus auf 10,7 % im 6. Einnahmezyklus. Während der Einnahme trat keine ungewollte Schwangerschaft auf. Von 101 Probandinnen wurde bei 57 Probandinnen jeweils am 8., 22., und 24. Zyklustag radioimmunologisch die Serumkonzentration von Progesteron gemessen. Nur bei einer einzigen Patientin wurde im 4. Einnahmezyklus ein Grenzwert von 4,0 ng/ml Progesteron gemessen. Alle anderen Meßwerte lagen deutlich unter 2 ng/ml. Damit konnte die sichere Ovulationshemmung bei Anwendung des erfindungsgemäßen Applikationsregime dokumentiert werden.

### Anwendungsbeispiel 2

- Tage 1 bis 3: 3 mg mikronisiertes 17β-Estradiol/d
- Tage 4 bis 7: 2 mg mikronisiertes 17β-Estradiol/d + 0,075 mg Desogestrel/d
- Tage 8 bis 23: 2 mg mikronisiertes 17β-Estradiol/d + 0,150 mg Desogestrel/d
- Tage 24 bis 25: 1 mg mikronisiertes 17β-Estradiol/d
- Tage 26 bis 28: Placebo

Bei dem zweiten Anwendungsbeispiel wurde gegnüber dem ersten Anwendungsbeispiel Estradiolvalerat durch mikronisiertes 17β-Estradiol ersetzt. An einer im Vergleich zu Beispiel 1 kleineren Anzahl von Probandinnen wurden analoge Ergebnisse erreicht.

### Anwendungsbeispiel 3

- Tage 1 bis 3: 2,5 mg konjugierte equine Estrogene (CEE)/d
- Tage 4 bis 7: 1,25 mg CEE/d + 1 mg Desogestrel/d
- Tage 8 bis 23: 1,25 mg CEE/d + 2 mg Desogestrel/d
- Tage 24 bis 25: 0,60 mg CEE/d
- Tage 26 bis 28: Placebo

In die entsprechende klinische Studie wurden 87 Frauen im Alter von 35-47 Jahren aufgenommen. Die Dauer der Einnahme betrug jeweils 6 Zyklen. Die Zwischenblutungsrate (Durchbruchsblutungen und Spottings) lag bei 15,7 % im ersten Einnahmezyklus und sank während der Einnahme kontinuierlich auf 7 % im letzten Einnahmezyklus.
Bei zusätzlich 17 Frauen wurde vor Beginn der Einnahme und am letzten Einnahmetag des 6. Studienzyklus ein oraler Glukosetoleranztest durchgeführt. Dabei waren in Hinblick auf die Insulinkonzentrationen keine Abweichungen vom Normbereich (2-25 mU/l) zu beobachten. Das Insulin/Glukose-Verhältnis war bei allen Probandinnen zu jedem Meßzeitpunkt im Normbereich. Der Anstieg des C-Peptid war signifikant. HbA1c zeigte keine signifikanten Veränderungen zwischen Kontrollzyklus und 6. Einnahmezyklus. Die Nüchternglukosewerte waren zur Abschlußuntersuchung bei 4 Probandinnen signifikant erhöht im Vergleich zum Kontrollzyklus. Im oralen Glukjosetoleranztest wiesen nur 3 Probandinnen eine eingeschränkte Glukosetoleranz auf. Zwei dieser 3 Probandinnen waren übergewichtig.
Die Ergebnisse belegen die überdurchschnittliche Stoffwechseltoleranz des erfindungsgemäßen Kontrazeptivums auf Basis natürlicher Estrogene.

### Anwendungsbeispiel 4

- Tage 1 bis 3: 2,5 mg konjugierte equine Estrogene (CEE)/d
- Tage 4 bis 7: 1,25 mg CEE/d + 150 mg mikronisiertes Progesteron/d
- Tage 8 bis 23: 1,25 mg CEE/d + 300 mg mikronisiertes Progesteron/d
- Tage 24 bis 25: 0,60 mg CEE/d
- Tage 26 bis 28: Placebo

Anstelle von Dienogest wird im Anwendungsbeispiel 4 als gestagene Komponente mikronisiertes Progesteron eingesetzt.

### Anwendungsbeispiel 5

- Tage 1 bis 3: 3 mg Estradiolvalerat/d
- Tage 4 bis 7: 2 mg Estradiolvalerat/d + 1 mg Dienogest/d
- Tage 8 bis 23: 2 mg Estradiolvalerat/d + 2,0 mg Dienogest/d
- Tage 24 bis 25: 1 mg Estradiolvalerat/d
- Tage 26 bis 28: Placebo

Die Resultate der in den Ausführungsbeispielen angesprochenen Studien belegen neben der kontrazeptiven Sicherheit auch die außerordentlich gute Zyklusstabilität des erfindungsgemäßen Applikationsregimes.

## Patentansprüche

1. Mehrphasenpräparat zur Kontrazeption
dadurch gekennzeichnet, daß
die erste Phase aus 2 bis 4 Tagesdosiseinheiten besteht, wobei jede Tagesdosiseinheit als Wirkstoff ausschließlich natürliche Estrogene enthält,
eine zweite Phase aus 2 Gruppen von Tagesdosiseinheiten einer Kombination aus mindestens einem natürlichen Estrogen und mindestens einem synthetischen oder natürlichen Gestagen besteht,
wobei die erste Gruppe aus 5 bis 3 Tagesdosiseinheiten und die zweite Gruppe aus 17 bis 13 Tagesdosiseinheiten gebildet wird,
eine dritte Phase aus 2 bis 4 Tagesdosiseinheiten besteht,
wobei jede Tagesdosiseinheit als Wirkstoff ausschließlich natürliche Estrogene enthält,
wobei die Tagesdosiseinheit an natürlichem Estrogen innerhalb der Phasen konstant bleibt, jedoch von Phase 1 zu Phase 3 abfällt
und der Anteil an synthetischen oder natürlichen Gestagen in der zweiten Gruppe der zweiten Phase den Anteil in der ersten Gruppe übersteigt
und eine weitere Phase aus 2 bis 4 Tagesdosiseinheiten besteht,
wobei jede Tagesdosiseinheit ein pharmazeutisch unbedenkliches Placebo enthält.

2. Mehrphasenpräparat nach Anspruch 1,
dadurch gekennzeichnet, daß
der estrogene Wirkstoff Estradiol, eine Estradiolverbindung aus der Estradiol nach Einnahme im Körper gebildet wird, ein konjugiertes equines Estrogen oder ein Phytoestrogen ist.

3. Mehrphasenpräparat nach Anspruch 1,
dadurch gekennzeichnet,
daß der gestagene Wirkstoff natürliches Progesteron, Medroxyprogesteronacetat oder ein synthetisches Gestagen ist.

## Claims

1. Multiphase preparation for contraception,
characterized in that
the first phase comprises 2 to 4 daily dose units,
each daily dose unit comprising, as the active compound, exclusively naturally occurring estrogens,
a second phase comprises 2 groups of daily dose units of a combination of at least one naturally occurring estrogen and at least one synthetic or naturally occurring gestagen,
the first group being formed from 5 to 3 daily dose units
and the second group being formed from 17 to 13 daily dose units,
a third phase comprises 2 to 4 daily dose units,
each daily dose unit comprising, as the active compound, exclusively naturally occurring estrogens,
the daily dose unit of naturally occurring estrogen remaining constant within the phases, but declining from phase 1 to phase 3
and the proportion of synthetic or naturally occurring gestagen in the second group of the second phase exceeding the proportion in the first group,
and a further phase comprises 2 to 4 daily dose units,
each daily dose unit comprising a pharmaceutically acceptable placebo.

2. Multiphase preparation according to claim 1,
characterized in that
the oestrogenic active compound is oestradiol, an oestradiol compound from which oestradiol is formed in the body after intake, a conjugated equine oestrogen or a phyto-oestrogen.

3. Multiphase preparation according to claim 1,
characterized in that
the gestagenic active compound is naturally occurring progesterone, medroxyprogesterone acetate or a synthetic gestagen.

## Revendications

1. Préparation contraceptive à plusieurs phases caractérisée en ce
la première phase se compose de 2 à 4 unités de doses quotidiennes, et chaque unité de dose quotidienne contient exclusivement, en tant qu'agent actif, des oestrogènes naturels,
une deuxième phase formée de 2 groupes d'unités de doses quotidiennes d'une combinaison d'au moins un oestrogène naturel et au moins un gestagène naturel ou synthétique,
et le premier groupe est formé de 5 à 3 unités de doses quotidiennes et le second groupe de 17 à 13 unités de doses quotidiennes,
une troisième phase se compose de 2 à 4 unités de doses quotidiennes et chaque unité de dose quotidienne contient comme agent actif exclusivement des oestrogènes naturels,
et l'unité de dose quotidienne de l'oestrogène naturel reste constante à l'intérieur des phases mais cependant baisse de la phase 1 à la phase 3,
et la proportion de gestagènes synthétiques ou naturels
dans le second groupe de la deuxième phase dépasse la proportion dans le premier groupe,
et une autre phase se compose de 2 à 4 unités de doses quotidiennes,
et chaque unité de dose quotidienne contient un placébo pharmaceutiquement inoffensif.

2. Préparation à plusieurs phases selon la revendication 1,
caractérisée en ce que
l'agent actif oestrogène est un estradiol, un composé d'estradiol qui est formé à partir de l'estradiol après absorption dans le corps, un oestrogène équin conjugué ou un phyto-oestrogène.

3. Préparation à plusieurs phases selon la revendication 1,
caractérisée en ce que
l'agent actif gestagène est la progestérone naturelle, l'acétate de médroxyprogestérone ou bien un gestagène de synthèse.
